# EUROPEAN PATENT SPECIFICATION

(11) **EP 1 231 934 B1**
(45) Date of publication and mention of the grant of the patent: **24.03.2004**
(21) Application number: 00980561.5
(22) Date of filing: 17.11.2000
(51) Int. Cl.: A61K 38/30, A61K 31/00, A61K 38/00, C12N 5/10, C12Q 1/02, A61P 3/00, A61P 3/08, A61P 21/00

(54) **METHODS OF INHIBITING ATROPHY OR PROMOTING HYPERTROPHY**
VERFAHREN ZUR HEMMUNG DER ATROPHIE ODER FÖRDERUNG DER HYPERTROPHIE
METHODES PERMETTANT D'INHIBER L'ATROPHIE OU DE FAVORISER L'HYPERTROPHIE

(30) Priority: 23.11.1999 US 167086 P
(43) Date of publication of application: 21.08.2002
(73) Proprietor: REGENERON PHARMACEUTICALS, INC., Tarrytown, NY 10591-6707 (US)
(72) Inventor: GLASS, David, J., Cortlandt Manor, NY 10567 (US); ROMMEL, Christian, Geneva CH-1208 (CH); BODINE, Sue, C., West Harrison, NY 10604 (US); YANCOPOULOS, George, D., Yorktown Heigths, NY 10598 (US)
(74) Representative: Duckworth, Timothy John
(86) International application number: PCT/US2000/031845
(87) International publication number: WO 2001/037852

(56) References cited:
- WO-A-00/20025
- WO-A-97/22360
- US-A- 5 444 047
- UEKI KOHJIRO ET AL: "Potential role of protein kinase B in insulin-induced glucose transport, glycogen synthesis, and protein synthesis." JOURNAL OF BIOLOGICAL CHEMISTRY, vol. 273, no. 9, 27 February 1998 (1998-02-27), pages 5315-5322, XP002169262 ISSN: 0021-9258
- COOLICAN SHARON A ET AL: "The mitogenic and myogenic actions of insulin-like growth factors utilize distinct signaling pathways" JOURNAL OF BIOLOGICAL CHEMISTRY,AMERICAN SOCIETY OF BIOLOGICAL CHEMISTS, BALTIMORE, MD,US, vol. 272, no. 10, 1997, pages 6653-6662, XP002161387 ISSN: 0021-9258 cited in the application
- COLEMAN M E ET AL: "MYOGENIC VECTOR EXPRESSION OF INSULIN-LIKE GROWTH FACTOR I SIMULATES MUSCLE CELL DIFFERENTIATION AND MYOFIBER HYPERTROPHY IN TRANSGENIC MICE" JOURNAL OF BIOLOGICAL CHEMISTRY,US,AMERICAN SOCIETY OF BIOLOGICAL CHEMISTS, BALTIMORE, MD, vol. 270, no. 20, 19 May 1995 (1995-05-19), pages 12109-12116, XP000611917 ISSN: 0021-9258 cited in the application
- ROMMEL CHRISTIAN ET AL: "Differentiation stage-specific inhibition of the Raf-MEK-ERK pathway by Akt" SCIENCE,AMERICAN ASSOCIATION FOR THE ADVANCEMENT OF SCIENCE,,US, vol. 286, no. 5445, 26 November 1999 (1999-11-26), pages 1738-1741, XP002161386 ISSN: 0036-8075

## Description

### BACKGROUND OF THE INVENTION

A decrease in muscle mass, or atrophy, is associated with various physiological and pathological states. For example, muscle atrophy can result from denervation due to nerve trauma; degenerative, metabolic or inflammatory neuropathy, e.g. Guillian-Barré syndrome; peripheral neuropathy; or nerve damage caused by environmental toxins or drugs. Muscle atrophy may also result from denervation due to a motor neuropathy including, for example, adult motor neuron disease, such as Amyotrophic Lateral Sclerosis (ALS or Lou Gehrig's disease); infantile and juvenile spinal muscular atrophies; and autoimmune motor neuropathy with multifocal conductor block. Muscle atrophy may also result from chronic disease resulting from, for example, paralysis due to stroke or spinal cord injury; skeletal immobilization due to trauma, such as, for example, fracture, ligament or tendon injury, sprain or dislocation; or prolonged bed rest. Metabolic stress or nutritional insufficiency, which may also result in muscle atrophy, include *inter alia* the cachexia of cancer and other chronic illnesses including AIDS, fasting or rhabdomyolysis, and endocrine disorders such as disorders of the thyroid gland and diabetes. Muscle atrophy may also be due to a muscular dystrophy syndrome such as Duchenne, Becker, myotonic, fascioscapulohumeral, Emery-Dreifuss, oculopharyngeal, scapulohumeral, limb girdle, and congenital types, as well as the dystrophy known as Hereditary Distal Myopathy. Muscle atrophy may also be due to a congenital myopathy, such as benign congenital hypotonia, central core disease, nemalene myopathy, and myotubular (centronuclear) myopathy. Muscle atrophy also occurs during the aging process.

Muscle atrophy in various pathological states is associated with enhanced proteolysis and decreased production of muscle proteins. Muscle cells contain lysosomal proteases and cytosolic proteases. The cytosolic proteases include Ca²⁺-activated neutral proteases (calpains) and an ATP-dependent ubiquitin-proteasome proteolytic system. The lysosomal and cytosolic systems are capable of degrading muscle proteins *in vitro,* but less is known about their roles in proteolysis of muscle proteins *in vivo.* Some studies have reported that proteasome inhibitors reduce proteolysis in atrophying rat skeletal muscle (e.g. Tawa et al. (1997) J. Clin. Invest. 100:197), leading to suggestions that the ubiquitin-proteasome pathway has a role in the enhanced proteolysis. However, the precise mechanisms of proteolysis in atrophying muscle remain poorly characterized.

Ras is a signaling molecule found in every cell in the body. It is activated by a variety of growth factors, including growth factors which signal tyrosine kinase receptors. Ras activation results in activation of several downstream signaling pathways, including the Raf/Mek/Erk pathway. Other potential downstream substrates of Ras include the following signaling molecules: PKC, Ral-GDS, KSR, and Rin-1 (Trends Genet 1999 Apr; 15(4):145). Ras, Raf, Mek and Erk genes are well-conserved through evolution, and homologs can be found in eukaryotes from yeast to humans. (Ras: Taparowsky E, et al. (1982) Nature 300:762; Raf: Heidecker, et al. (1990) Mol. Cell. Biol. 10:2503; Samuels, et al. (1993) Mol. Cell. Biol. 13:6241; Morrison, et al. (1997) Curr. Opin. Cell Biol. 9:174; Mek: Crews CM, et al. (1992) Science 258:478; Erks: Boulton TG, et al. (1991) Cell 65 :663).

Another pathway that is often simultaneously activated in response to the same growth factors and hormones as the Ras/Raf/Mek/Erk pathway is the PI3 kinase (phosphatidylinositol 3-kinase)/Akt pathway (PI3K/Akt). (Vanhaesebroeck, et al. (1997) TIBS 22:267; Toker and Cantley (1997) Nature 387:673; Rameh and Cantley (1999) J. Biol. Chem. 274:8347). In some systems, the small guanine nucleotide binding protein, Ras, acts as an upstream positive effector of both the Raf/Mek/Erk pathway and the P13 /Akt pathway. (Katz, et al. (1997) Curr. Opin. Genet. Dev. 7:75; Rodriquez-Viciana et al. (1997) Cell 89:457; Rommel, et al. (1998) Curr. Opin. Genet. Dev. 4:412). In fact, IGF-1, which causes hypertrophy in myotubes *in vitro,* has been shown to activate both the Ras/Raf/Mek/Erk pathway and the PI3K/Akt pathway. (Quinn, et al. (1994) J. Cell Physiol. 3:387; Coleman, et al. (1995) J. Biol. Chem. 270:12109; Collican, et al. (1997) J. Biol. Chem. 272:6653; Skolnik, et al. (1993) Science 260:1953; Florini, et al. (1996) Endocr. Rev. 17:481; Stokoe, et al. (1997) EMBO-J 16:2384; Avruch (1998) Mol. Cell. Biochem. 182:31). However, it has also been proposed that these two pathways can have opposing effects. Manipulation of these pathways during muscle differentiation indicates that inhibition of the Ras/Raf/Mek/Erk pathway can enhance differentiation, and that inhibition of P13/Akt pathway blocks differentiation. (Bennet, et al. (1997) Science 278:1288; Coolican, et al. (1997) J. Biol. Chem. 272:6653; Jiang, et al. (1998) Proc. Natl. Acad. Sci. 24:14179). However, there has been no evaluation of the roles of these two pathways in the process of skeletal muscle atrophy and/or hypertrophy.

Studies have shown that in response to increases in external load, skeletal muscle adapts by increasing muscle mass and protein content primarily through an increase in muscle fiber size which translates into greater tension output (Carson, J.A. Exercise Sport Science Rev. 25: 301-320 (1997)). However, the signaling pathways responsible for the regulation of muscle fiber size and muscle mass are unknown. Initial studies in cardiac hypertrophy (Molkentin, J.D. *et al. Cell* 93:215-228 (1998)) as well as early studies with skeletal muscle cells *in vitro* (Molkentin, J.D. *et al*. *Cell* 93:215-228 (1998); (Semarian, C. *et al. Nature* 400: 576-581 (1999) pointed toward a key role for the cyclosporin-inhibitable phosphatase known as calcineurin. However, recent attempts to determine the effects of calcineurin manipulation on skeletal muscle hypertrophy *in vivo* have been inconclusive (Semarian, C. *et al. Nature* 400: 576-581 (1999); Musaro, A. *et al Nature* 400: 581-585 (1999)). Recently, Baar and Esser (Baar, K. & Esser, K. Am. J. Physiol. Cell 45: C120-C127 (1999)) showed that phosphorylation of the 70-kD S6 protein kinase (p70^{S6K}) increases in muscles with hypertrophy following a high-resistant exercise training protocol in rats. Additionally, IGF-1, which activates the PI3K/Akt pathway, is upregulated during load-induced hypertrophy (Carson, J.A. Exercise Sport Science Rev. 25: 301-320 (1997); Adams, G.R. & Haddad, G.R. J. Appl. Physiol. 81: 2509-2516 (1996)) and leads to muscle hypertrophy in transgenic mice overexpressing IGF-1 under the control of a skeletal-actin promoter (Coleman, M.E. *et al* J. Biol. Chem. 270: 12109-12116 (1995)). These observations are intriguing because p70^{S6K} and the 4E binding protein (PHAS-I) are key phosphoproteins involved in the regulation of protein translation initiation (Brunn, G.J. *et al*. Science 277: 99-101 (1997); Rhoads, R.E. J. Biol. Chem. 274: 30337-30340 (1999)). Moreover, activation of p70^{S6K} correlates to increases in the translation of mRNA containing a 5'-TOP tract, including ribosomal proteins and the elongation factors (Vary, T.C. *et al*. Am. J. Physiol. Endocrinol. Metab. 278: E58-E64 (2000)). Phosphorylation of PHAS-I leads to its dissociation from eIF4E allowing eIF4E to bind to the 5'-cap structure of an mRNA and initiate the formation of the eIF4F complex. The phosphorylation/activation of p70^{S6K} and PHAS-I is regulated, in part, by a protein kinase, mTOR which is activated by the PI3K/Akt pathway and inhibited by rapamycin.

Therefore, in accordance with the present invention, it has been discovered that activation of the PI3K/Akt pathway results in increased hypertrophy and blocking of atrophy of skeletal muscle cells.

### SUMMARY OF THE INVENTION

The present invention provides a method of identifying a test agent that inhibits muscle atrophy comprising:
(a) obtaining cells that express the following:
   1) Akt;
   2) an Akt reporter substrate capable of measuring Akt substrate activation;
   3) Raf; and
   4) a Raf reporter substrate capable of measuring Raf substrate activation;
(b) subjecting the cells to a test agent;
(c) measuring the amount of Akt substrate activation in (a)(2);
(d) measuring the amount of Raf substrate activation in (a)(4),
wherein activation of the Akt substrate absent activation of the Raf substrate is used to identify a test agent that inhibits muscle atrophy.

Agents that activate the PI3K/Akt pathway, which in turn block Raf activity, and thereby inhibit the Raf/Mek/Erk pathway, are useful for preventing or reducing atrophy and/or causing hypertrophy in skeletal muscle cells.

In preferred embodiments of the method the cells are fibroblasts, muscle cells, myoblasts, or C2C12 cells.

Another embodiment is a cell comprising:
1) Akt;
2) an Akt reporter substrate capable of measuring Akt substrate activation;
3) Raf; and
4) a Raf reporter substrate capable of measuring Raf substrate activation.

### BRIEF DESCRIPTION OF THE FIGURES

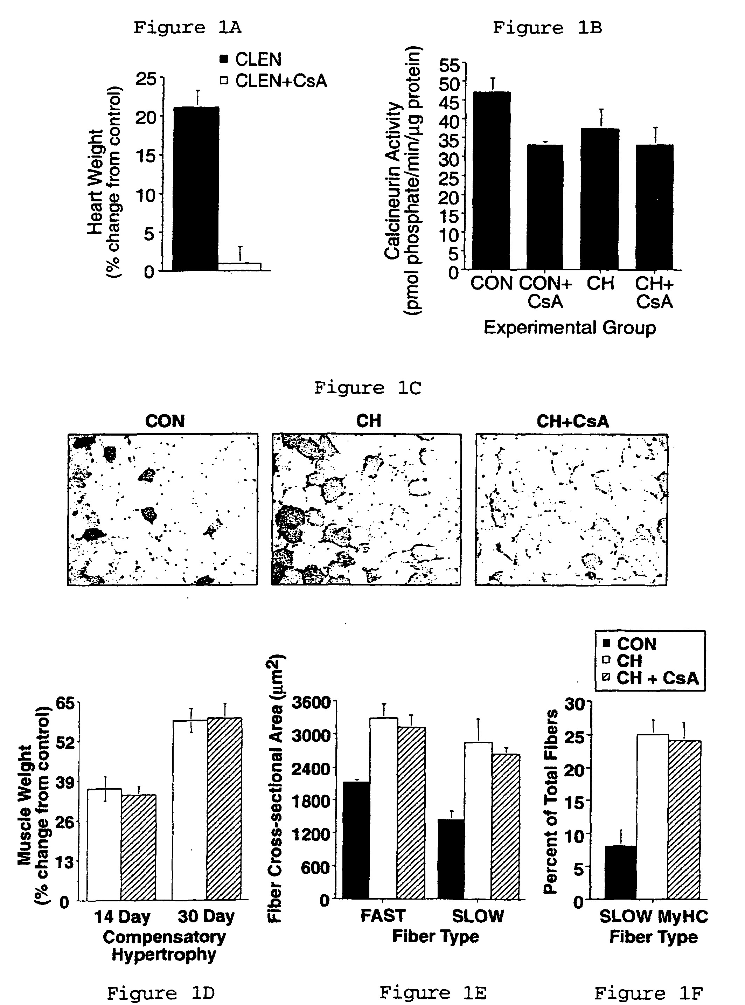

**Figure 1A-1F: Muscle hypertrophy is not blocked by cyclosporin A.** Figure 1A: Weight of the rat heart, expressed as a percent change from control, following daily treatment with the b₂-adrenergic agonist denbuterol (3 mg/kg, s.c.) for 14 days (CLEN) or daily treatment with denbuterol and cyclosporin for 14 days (CLEN+CsA) (10 rats/group). Figure 1B: Calcineurin phosphatase activity measured in plantaris muscle lysates from control rats (CON), control rats treated with cyclosporin for 4 days (Con+CsA), 4 day compensatory hypertrophy rats (CH), and 4 day CH rats treated with cyclosporin (CH+CsA). Total amount of calcineurin was similar between groups as measured by Western blot. Figure 1C: Cross-sections of the rat plantaris muscle were stained with an anti-MyHC slow antibody. Groups were control (CON), 14d compensatory hypertrophy (CH) and 14d CH treated daily with cyclosporin (CH+CsA). Figure 1D: Weight of the rat plantaris muscle, expressed as a percent change from control, following 14 or 30 days of compensatory hypertrophy with vehicle (CH) or cyclosporin treatment (CH+CsA) (10 rats/group). Figure 1E: Cross-sectional area of muscle fibers in the rat plantaris muscle of control (CON), 14 day compensatory hypertrophy (CH) or 14 day compensatory hypertrophy plus cyclosporin treatment (CH+CsA) (5 rats/group). Muscle fibers were classified as slow or fast based on staining with an anti-MyHC-slow antibody. Figure 1F: Percentage of muscle fibers expressing slow myosin heavy chain in the plantaris muscle of control (CON), 14 day compensatory hypertrophy (CH) or 14 day compensatory hypertrophy plus cyclosporin treatment (CH+CsA) (5 rats/group).

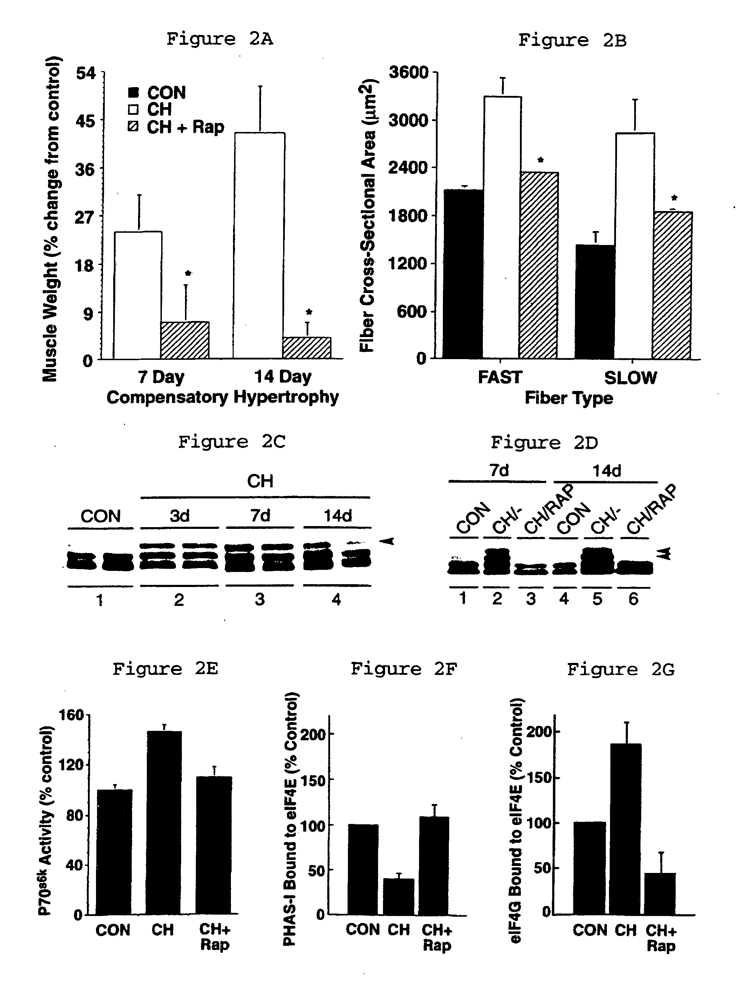

**Figure 2A-2G: Muscle hypertrophy is associated with increased phosphorylation of p70**^{**s6k**} **and is blocked by rapamycin.** Figure 2A: Weight of the rat plantaris muscle, expressed as a percent change from control, following 7 or 14 days of compensatory hypertrophy with vehicle (CH) or rapamycin treatment (CH + Rap) (10 rats/group). Asterisk indicates significant difference from CH group (p<0.05). Figure 2B: Cross-sectional area of muscle fibers in the rat plantaris muscle of control (CON), 14 day compensatory hypertrophy (CH) or 14 day compensatory hypertrophy plus rapamycin treatment (CH+Rap) (5 rats/group). Muscle fibers were classified as slow or fast based on their staining with an anti-MyHC-slow antibody. Asterisk indicates significant difference from CH group (p<0.05). Figure 2C: Western blots demonstrated an increase in the phosphorylation of p70^{s6k} in the plantaris during compensatory hypertrophy (CH) as evidenced by notable gel shifts. Each lane represents 25 mg of total protein extracted from a pool of 3 plantaris muscles after control (CON, lanes 1), 3d CH (lanes 2), 7d CH (lanes 3), or 14d CH (lanes 4). Arrowhead indicates hyperphosphorylated p70^{s6k} species. For each group, duplicate lanes represent different pools of plantaris muscles. Figure 2D: Western blots of p70^{s6k} in the rat plantaris. The p70^{s6k} gel shift observed after 7 (lane 2) and 14 (lane 5) days of compensatory hypertrophy (CH/-) was inhibited by daily injections of rapamycin (CH/RAP; lanes 3 and 6). Figure 2E: The specific activity of p70^{s6k} was determined by ³²P incorporation into 40s ribosomes in the immune complex. The p70^{s6k} activity was measured in the plantaris after 14d of compensatory hypertrophy (CH) and after 14d treated with rapamycin (CH+Rap). Figure 2F: PHAS-I bound to eIF4E after 14d compensatory hypertrophy (CH) or 14d CH plus rapamycin (CH+Rap). After correction for the amounts of eIF4E recovery, the results were expressed as a percentage of the respective controls and are the means ± the range of two experiments. Increased phosphorylation of PHAS-I leads to its disassociation from the initiation factor eIF4E. The percentage of PHAS-I bound to eIF4E was significantly lower than control following CH. Treatment of CH rats with rapamycin inhibited the phosphorylation of PHAS-I and prevented the disassociation of PHAS-I from eIF4E. Figure 2G: eIF4G bound to eIF4E after 14d compensatory hypertrophy (CH) or 14d CH plus rapamycin (CH+Rap). After correction for the amounts of eIF4E recovery, the results were expressed as a percentages of the respective controls and are the means ± the range of two experiments. The percentage of eIF4E bound to eIF4G was significantly increased from control following CH. Treatment of CH rats with rapamycin significantly decreased the percentage of eIF4E bound to eIF4G.

### DETAILED DESCRIPTION OF THE INVENTION

Muscle atrophy can be caused by the enhanced proteolysis of muscle proteins, resulting in a decrease in muscle mass. In addition, muscle atrophy can be caused by a diminished synthesis of proteins, resulting in a decrease in muscle mass.

It has been discovered in accordance with the present invention that when skeletal muscle cells contain and express an activated mutant form of the Akt transgene, the resultant cells appear hypertrophic in comparison to control skeletal muscle cells.

It has also been discovered in accordance with the present invention that activation of the PI3K/Akt pathway inhibits the Raf/Mek/Erk pathway in differentiated myotubes. Thus, the phenotype observed using constitutively active Akt is similar, although more pronounced, than that observed when the dominant negative form of Raf is expressed. It has been previously discovered by Applicants that when skeletal muscle cells contain and express a "dominant negative" mutant form of the Raf transgene, the resultant cells appear hypertrophic in comparison to control cells. A "dominant negative" mutant form of a signaling molecule is a mutant which is capable of inhibiting normal signaling of that molecule.

Accordingly, Applicants have discovered that specific activators of the PI3K/Akt pathway are useful for reducing or preventing atrophy or causing hypertrophy in skeletal muscle cells. As used here, "specific activators" of the PI3K/Akt pathway are those which result in activation of a substrate within the PI3K/Akt pathway, but which do not activate the Ras/Raf/Mek/Erk pathway. For example, IGF would not be considered a specific activator of the PI3K/Akt pathway, as defined herein, since it also activates the Ras/Raf/Mek/Erk pathway. In a preferred embodiment, the specific activator is an activator of Akt. In additional preferred embodiments, the specific activator is an activator of mTOR, p70^{S6K}, or PHAS-I.

Specific activators are agents that may be used to decrease and/or prevent atrophy in mammals having a condition, such as those described herein, in which skeletal muscle atrophy is occurring. Atrophying skeletal muscle cells, or vertebrate animals having a condition as described above in which muscle cells are atrophying, are treated with a specific activator so as to prevent or decrease muscle cell atrophy. Such treatment may be utilized prophylactically prior to the onset of muscle atrophy or after such condition has manifested itself. Vertebrate animals include any species containing skeletal muscle and a backbone, and includes chickens, rodents, rabbits, dogs, cats, cows, horses, pigs, sheep, primates, and humans, preferably humans.

Specific activators of the PI3K/Akt pathway are agents that may be used to cause hypertrophy in skeletal muscle cells. Further, such specific activators may be used to cause muscle hypertrophy in vertebrate animals having conditions, such as those described herein, in which skeletal muscle atrophy is anticipated. In some settings, such as in animals farmed for meat production, such agents might be used to increase meat production.

Therapeutic compositions. comprising a specific activator of the PI3K/Akt pathway in a carrier which may include excipients, diluents or other compounds. Such compositions may be administered systemically or locally. Any appropriate mode of administration known in the art may be used including, but not limited to, intravenous, intrathecal, intraarterial, intranasal, oral, subcutaneous, intraperitoneal, or by local injection or surgical implantation. Sustained release formulations are also provided for.

The activity of such compositions in vertebrate animals may be assessed using experimental animal models of disorders in which muscle atrophy is present. For example, the activity of the compositions may be tested for their effect in the hindlimb immobilization model described herein in Example 2 *infra*. Alternatively, the activity of the compositions may be assessed using experimental animals in which hypertrophy can be measured. For example, the activity of the compositions may be tested for their effect on muscles undergoing exercise-induced hypertrophy, or compensation-induced hypertrophy (see Example 3 *infra*). Alternatively, the muscle may be assessed in control animals as compared to animals treated with the experimental compositions, to determine if the treated animals exhibit skeletal muscle hypertrophy as a result of their treatment. Data obtained from cell culture assays and animal studies can be used in formulating a range of dosages for use in vertebrate animals, including humans. The dosage of the compositions of the invention should lie within a range of serum circulating concentrations with little or no toxicity. The dosage may vary within this range depending on the dosage form employed and the route of administration.

To screen for specific activators of the PI3K/Akt pathway in a cell-based assay, a cell which expresses Akt, such as a myoblast or myotube, but also any other cell which expresses Akt, including a fibroblast, may be used to determine if a reporter substrate containing an Akt phosphorylation site or downstream component ("Akt substrate/reporter construct") becomes phosphorylated when contacted with a test agent. One skilled in the art will recognize that other components in the PI3K/Akt pathway may also be used in place of Akt, provided the appropriate reporter substrate is present. For example, the assay may comprise PI3K and a substrate/reporter construct that comprises Akt. Examples of substrates that are phosphorylated by Akt include GSK3 (glycogen synthase kinase 3) (Cross, et al. (1995) Nature 378: 785, BAD; Datta, et al. (1997) Cell 91,231-41; del Peso, et al. (1997) Science 278: 687), the transcription factor "forkhead" (Brunet, (1999) Cell 96: 857; Kops, et al. (1999) Nature 398: 630); and mTOR.

To assess Akt-specificity, other reporter substrates activated, for example, by a component of the Ras/Raf/Mek/Erk pathway, or downstream molecules that are activated by this pathway (Raf reporter substrates), are followed to assure that this pathway is not activated by the agents identified as described herein which activate the PI3K/Akt pathway. Examples of reporter constructs that may be used to assess contact between Akt and a substrate include those used in FRET-based assays.
Activation, as used herein, would be defined as an amount above the level normally expressed by the cell in the absence of the agent. A good review of the signaling components which are part of the Ras/Raf/Mek/Erk pathway, as well as downstream components effected by this pathway, is contained in Rommel and Hafen (1998) Curr. Opin. Genet. Devel. 8:412.

According to the invention, cells are created that comprise Akt, an Akt reporter substrate capable of measuring Akt substrate activation, Raf, and a Raf substrate/reporter construct capable of measuring Raf substrate activation. Such cells are subjected to a test agent, and the amount of Akt substrate activation and the amount of Raf substrate activation are measured using the respective reporter substrates. Test agents that may be utilized to inhibit atrophy or cause hypertrophy are identified as those that cause Akt substrate activation without causing Raf substrate activation. The present invention also contemplates cells that express Akt as well as Raf/Mek/Erk signaling components and the respective reporter constructs that may be used to determine activation of Akt and Raf/Mek/Erk in the cells.

Cells useful for expressing Akt and Raf and their associated reporter substrates include non muscle cells, as well as any and all muscle cells that can be maintained in culture and that can be engineered to express a heterologous nucleic acid. The cells may be primary cultures or established cell lines. Suitable muscle cells include myoblasts, for example the C2C12 cell line as described in Bains, et al. (1984) Mol. Cell. Biol. 4:1449, the disclosure of which is incorporated herein by reference. Other suitable muscle cells include Sol8 cells, described by Glass et al. (1997) Proc. Natl. Acad. Sci. USA 16:8848, and L6 cells, described by Ringentz et al. (1978) Exp. Cell Res. 113:233.

The Akt, Raf and reporter substrate nucleic acids under the control of suitable transcriptional and translational regulatory sequences can be introduced into the cell by methods known in the art including, for example, transformation, transfection, infection, transduction and injection. The expression vector containing Akt and Raf reporter substrate nucleic acids under the control of suitable promoters is introduced into cells by known methods, for example liposome-mediated transfection, calcium phosphate-mediated transfection, DEAE-dextran transfection, naked DNA transfection, microinjection, electroporation, retroviral-mediated infection, adenoviral-mediated infection, or adeno-associated viral-mediated infection. The reporter construct nucleic acids can be introduced into the cell stably or transiently. Methods for introducing heterologous nucleic acids into eukaryotic cells are described in numerous laboratory manuals including, for example, DNA Cloning: A Practical Approach, vols. I-III (1985) Glover, ed., IRL Press Limited, Oxford, and Sambrook et al. (1989) Molecular Cloning: A Laboratory Manual, 2^{nd} edition, Cold Spring Harbor, NY.

In a preferred embodiment, the nucleic acid is inserted into a retroviral vector, for example, as described by Pear et al. (1993) Proc. Natl. Acad. Sci. USA 90:8392, incorporated herein by reference. In this embodiment, the viral LTR promoter controls the transcription of the nucleic acid. The vector is transiently transfected into a retroviral packaging line, and the resulting recombinant virus which contains the nucleic acid is harvested, as described by Pear et al., id. The recombinant virus is then used to infect myoblasts as described by Hoffman et al. (1996) Proc. Natl. Acad. Sci. USA 93:5185, incorporated herein by reference. C2C12 cells, as an example of skeletal muscle cells expressing Akt, can be maintained in the undifferentiated state by growing them in tissue culture media containing at least 10% fetal calf serum, or they can be differentiated into skeletal muscle myotubes by growing them in media containing 2% horse serum. The necessary tissue culture methods are known to those of ordinary skill in the art. C2C12 cells are described in Bains et al. (1984) Mol. Cell Biol. 4:1449.

In methods for the identification of an agent that inhibits muscle cell atrophy, or causes hypertrophy, the agent may be contacted with the cell comprising Akt, Raf and the reporter substrates constructs by methods known in the art. For cells in culture or cells obtained from transgenic organisms, the cell may be contacted with the agent by, for example, direct application. The agent may be modified or contained in a delivery vehicle to facilitate entry into the cell. The agent may be isolated and purified, or it may be present in a sample or composition to be subjected to further isolation and purification subsequent to a positive result in the present method. For example, the agent may be contained in a cell lysate, conditioned cell culture media, or a library of synthetic or naturally occurring compounds. For muscle cells present in a transgenic organism, the cells may be contacted with the agent by delivering the agent by methods known in the art, for example by ingestion, parenteral administration, or direct application to tissue surfaces, and may be present in a composition comprising a carrier or diluent. Agents that may be tested in the method of the present invention include, for example, organic and inorganic molecules such as proteins, peptides, lipids, carbohydrates, nucleic acids, including antisense, metals, salts, and so on.

Test agents identified above may be assessed for their ability to cause hypertrophy or reduce atrophy in cultured muscle cells. The amount of atrophy in cells may be measured by quantitation of cell diameter, protein amount, or by activation of the PHAS-I protein or p70^{S6K} which stimulate protein synthesis. In vertebrate animals, muscle atrophy may be measured as described in Example 2 herein.

The presence of activated PI3K/Akt and Raf/Mek/Erk pathway substrates can also be assessed using antibodies specific for these proteins.

In another embodiment, the present invention provides a method of identifying an agent that inhibits atrophy in muscle cells, comprising preparing an *in vitro* assay for Akt, contacting the Akt with an agent to be tested; and screening for agents which activate the Akt protein. According to this embodiment, an assay could be developed to take advantage of the fact that Akt is a kinase, and when activated it phosphorylates serine and threonine on specific substrates. Thus, Akt protein could be contacted with an appropriate substrate and a test agent, in an *in vitro* kinase assay, and the substrate could then be analyzed for whether it had become phosphorylated by Akt. To assure specificity, other serine- threonine protein kinases, such as Raf, would also be assayed with the same test agent, to assure that the agent causes activation of Akt specifically.

The methods of the present invention are useful for the identification of agents that prevent atrophy or cause hypertrophy in muscle cells by specifically activating the PI3K/Akt pathway. The agents identified by the present methods are useful for the treatment and prevention of muscle atrophy and for causing muscle hypertrophy.

In addition to the embodiments described *supra,* a tissue-specific mechanism of activating the PI3K/Akt pathway would also be very valuable. Applicants have demonstrated that activation of PI3K can lead to an increase in protein synthesis, and can block muscle atrophy. Current agents such as IGF-1 activate the PI3K/Akt pathway in skeletal muscle, but also in many other tissues, since the receptor for IGF-1 is ubiquitous. Therefore, an agent whose receptor is tissue-specific, or which can be engineered to act in a tissue-specific fashion, such as by specific introduction of the agent into muscle, or by joining the agent to a second moiety which confers tissue specificity, would be useful in blocking muscle atrophy, or in inducing muscle hypertrophy. A tissue-specific activator of the PI3K/Akt pathway would have the additional benefit of avoiding side-effects caused by lack of specificity, such as cardiac muscle hypertrophy, for example. A tissue-specific mechanism of activating the PI3K/Akt pathway would not need to be absolutely specific for the PI3K/Akt pathway. It has been shown that IGF-1, which activates the PI3K/Akt pathway, also simultaneously activates the Ras/Raf pathway. However, in skeletal muscle, Akt inhibits Raf, as shown in the Example 1 infra; therefore, the activation of the Ras/Raf pathway would not interfere with the mechanism of a skeletal muscle-specific activator of PI3K/Akt.

The following non-limiting examples serve to further illustrate the present invention.

### EXAMPLES

### Example 1: The effect of activation of AKT in muscle cells.

The role of the PI3K/Akt pathway in C2C12 myoblasts and differentiated myotubes was examined by genetic manipulation. Genetic manipulation of C2C12 cells was accomplished by the transfection of vectors capable of expressing a gene of interest as well as the green fluorescent protein (GFP) reporter gene. This enabled isolation of rare clones of transfected cells expressing desired levels of the gene of interest, using standard fluorescence activated cell sorter (FACS) technology. Expression of the transgenes was confirmed by standard immunoblotting. HA-epitope tagged constitutively active Akt (Franke, et al. (1995) Cell., 81:2143) and constitutively active PI3K (Khwaja, et al. (1998) J. Biol. Chem. 273:18793) were subcloned into a bi-cistronic vector consisting of the MCK promoter (Jaynes, et al. (1988) Mol. Cell. Biol. 8:62 and an IRES-EGFP cassette (Clontech). Subconfluent C2C12 myoblasts were transfected by calcium phosphate-mediated co-transfection (Specialty Media, Inc.). Transfection was performed as described previously (Glass, et al. (1996) Cell 85:513). Flow cytometry and cell sorting were carried out on a Cytomation MoFlo (Fort Collins, CO) high speed cell sorter. Laser excitation was 130 mw at 488 nm. Fluorescence emission was collected through a 530/540 nm band pass filter for GFP. For sorting, cells were collected at a sort rate of 25,000 cells/second. The collected myoblasts were then grown to confluence, and differentiated by standard techniques into "myotubes", which are multi-nucleated muscle cells, similar to actual muscle fibers.

Expression of GFP alone did not alter the C2C12 differentiation process. Vector-driven expression of a constitutively active form of Akt (Bellacosa, et al. (1991) Science 254:274; Kohn, et al. (1996). J. Biol. Chem. 271:31372; Eves, et al. (1998) Mol. Cell. Biol. 18:2143) caused a hypertrophic phenotype, resulting in thickened but shortened multinucleated myotubes. The effect was even more pronounced than that observed previously by the Applicants, in which a dominant negative form of Raf is expressed by C2C12 cells. Thus, genetic manipulation of the Raf/Mek/Erk and the PI3K/Akt pathways revealed opposing phenotypic effects during the muscle differentiation process, with the Raf/Mek/Erk pathway inhibiting the hypertrophic phenotype while the PI3K/Akt pathway promoting the hypertrophic phenotype. This phenotypic correlation was confirmed by examining expression of myogenin and p21CIP, two markers of myoblast differentiation. While constitutively active Raf decreases their expression, dominant negative Raf and constitutively active Akt increased the expression of both of these markers.

Biochemical evaluation of the Raf/Mek/Erk and PI3K/Akt pathways in the genetically manipulated myotubes revealed cross-regulation between the two pathways. Erk phosphorylation was used as a downstream marker of the Raf/Mek/Erk pathway activation. Erk phosphorylation was induced in C2C12 myotubes by serum and an unrelated factor, heregulin-β. In addition, Erk was constitutively phosphorylated in myotubes expressing constitutively active Raf. In contrast, Erk phosphorylation induced by serum and heregulin-β was significantly inhibited in myotubes in which the PI3K/Akt pathway was constitutively activated by expressing constitutively active Akt. To confirm these findings, a constitutively active form of PI3K (Klippel, et al. (1996) Mol. Cell. Biol. 16:4117; Khwaja, et al. (1998) J. Biol. Chem. 273:18793) was expressed, to activate endogenous Akt in differentiated C2C12 myotubes (as verified by increased levels of endogenous Akt phosphorylation). As observed in myotubes expressing constitutively active Akt, the level of phosphorylation of Erk was similarly inhibited. Thus, activation of the PI3K/Akt pathway inhibited activation of Erk. Although almost complete ablation of serum-induced activation of Erk in differentiated C2C12 myotubes expressing constitutively active Akt was observed, no inhibition of Erk activation occurred in myoblasts, despite similar levels of expression of the constitutively active Akt. Thus, the regulatory effect of Akt on the Erk pathway is specific to differentiated muscle cells.

The mechanism for the stage-specific inhibition of the Raf/Mek/Erk pathway by Akt was addressed by examining Raf /Akt complex formation. Expression of endogenous Raf was comparable in myoblasts and myotubes. Constitutively active Akt could be co-immunoprecipitated with Raf from different myotubes, but not from myoblasts. A kinase-inactive form of Akt did not associate with Raf in differentiated myotubes, consistent with the notion that neither Akt activation or membrane localization was required for its association with Raf in myotubes.

Akt can phosphorylate Raf *in vitro,* but the cross-regulatory mechanism identified herein cannot simply be explained by obligate binding of Akt to Raf, followed by Akt phosphorylation of Raf, since both binding and cross-regulation occur in myotubes but not myoblasts. Thus, Akt and Raf do not obligately interact. Regulation of Raf/Akt interactions may involve stage-specific modifications of these proteins, or stage-specific proteins that regulate formation of Raf/Akt complexes.

### Example 2: Animal model for atrophy.

To test for muscle atrophy, the ankle joint of rodents (mice or rats) are immobilized at 90 degrees of flexion. This procedure induces atrophy of the muscles with action at the ankle joint (e.g. soleus, medial and lateral gastrocnemius, tibilias anterior) to varying degrees. A reproducible amount of atrophy can be measured in hindlimb muscles over a 14-day period..

The immobilization procedure may involve either casting (mice) or pinning the ankle joint (rats). Rodents are anesthetized with ketamine/xylazine and the right ankle joint is immobilized. In rats, a 0.5 cm incision is made along the axis of the foot, over the heel region. A threaded screw (1.2 x 8mm) is then inserted through the calcaneous and talis, into the shaft of the tibia. The wound is closed with skin glue. In mice, the ankle joint is fixed at 90 degrees with a light weight casting material (VET-LITE) around the joint. The material is soaked in water and then wrapped around the limb. When the material dries it is hard, but light in weight.

At seven and 14 days following the immobilization, animals are anesthetized and killed by cervical dislocation. The tibialis anterior (TA), medial gastrocnemius (MG), and soleus (Sol) muscles are removed from the right (immobilized) and left (intact) hindlimbs, weighed, and frozen at a fixed length in liquid nitrogen cooled isopentane. A cohort of control animals which are the same weight and age as the experimental animals are also killed and the muscles removed, weighed and frozen. The amount of atrophy is assessed by comparing the weight of the muscles from the immobilized limb with the weight of the muscles from the control animals. Further assessment of atrophy will be done by measuring muscle fiber size and muscle tension output.

### Example 3: Muscle hypertrophy is associated with increased phosphorylation of p70^{S6K} and PHAS-I and is blocked by rapamycin.

The roles of the calcineurin and mTOR pathways in producing muscle hypertrophy were examined in the compensatory muscle hypertrophy model. When a fast-twitch skeletal muscle is subjected to a chronic workload increase by removing functionally synergistic muscles, the muscle compensates by increasing fiber size and muscle mass, as well as by switching fibers to a slow-twitch phenotype (Adams, G.R. & Haddad, G.R. *J. Appl. Physiol*. 81: 2509-2516 (1996).; Roy, R.R. *et al. J. Appl. Physiol.* 83: 280-290 (1997)). Applicants induced functional overload of the rat plantaris muscle by surgically removing the soleus and gastrocnemius muscles. Cyclosporin A (CsA) was given at a dosage (15 mg/kg, s.c.) that was sufficient to block pharmacologically-induced cardiac hypertrophy using the β₂- adrenergic agonist, denbuterol (Figure 1A) and inhibited calcineurin activity in control skeletal muscle (Figure 1B). Treatment with CsA was unable to prevent compensatory hypertrophy of the plantaris at 7, 14 or 30 days following the surgical overload as evidenced by the increases in muscle mass (Figure 1D) and fiber size (Figure 1C & Figure 1E). In contrast, rapamycin treatment almost completely prevented the increases in plantaris muscle mass (Figure 2A) and fiber size (Figure 2B) in both rats and mice at 7 and 14 days following the removal of synergists. Rapamycin treatment did not exhibit non-specific effects since it had no effect on body weight or the baseline mass of non-hypertrophying hindlimb muscles in these adult animals. Further, rapamycin given to control adult animals for 14 days had no effect on body weight or muscle mass. Consistent with these findings suggesting a requisite role for the PI3K/Akt/mTOR pathway and not the calcineurin pathway in compensatory muscle hypertrophy, downstream targets of mTOR - p70^{S6K} and PHAS-I - were inducibly phosphorylated (as evidenced by notable gel shifts for p70^{S6K}, Figure 2C and for PHAS-I) and activated (in terms of kinase activity for p70^{S6K}, Figure 2E, and in terms of release of PHAS-I, from it sequestration within an inhibitory complex with the translation initiation factor eIF4E, Figure 2F, thereby allowing binding of eIF4E to eIF4G, Figure 2G) immediately upon the initiation, as well as during the entire duration of the hypertrophic response. In contrast, calcineurin activity was not increased in hypertrophying muscle, and in fact was decreased (Figure 1B). Moreover, just as rapamycin treatment completely blocked the hypertrophic response, it also completely blocked the accompanying binding and activation of p70^{S6K} (Figure 2D & Figure 2E) and PHAS-I (Figure 2F & Figure 2G) in hypertrophying muscle.

Applicants' findings demonstrate that adaptive hypertrophy of adult skeletal muscle is not dependent on a calcineurin signaling pathway, but rather appears to be critically regulated by the activation of the PI3K/Akt/mTOR pathway and its downstream targets, p70^{S6K} and PHAS-I. While Applicants' data reveal that maintenance of muscle mass in mature animals is not dependent on the mTOR pathway (since rapamycin did not produce a loss of muscle mass in adult rodents), this pathway appears critical in load-induced hypertrophy of adult muscle and recovery of mass following atrophy.

### Example 4: The effect of inhibiting GSK3 (Glycogen Synthase Kinase 3) in skeletal muscle cells.

The role of the PI3K/Akt/GSK3 pathway in C2C12 myoblasts and differentiated myotubes was examined by genetic manipulation. Genetic manipulation of C2C12 cells was accomplished by the transfection of vectors capable of expressing a gene of interest as well as the green fluorescent protein (GFP) reporter gene. This enabled isolation of rare clones of transfected cells expressing desired levels of the gene of interest, using standard fluorescence activated cell sorter (FACS) technology. Expression of the transgenes was confirmed by standard immunoblotting. Akt phosphorylation of GSK3 results in inhibition of GSK3 activity. Therefore, to model the effect of GSK3 inhibition, a dominant-negative, kinase dead, HA-epitope tagged mutant of GSK3 (Dominguez and Green, Development, 2000, v 127, p861-868)) was subcloned into a bi-cistronic vector consisting of the MCK promoter (Jaynes, et al. (1988) Mol. Cell. Biol. 8:62 and an IRES-EGFP cassette (Clontech). Subconfluent C2C12 myoblasts were transfected by calcium phosphate-mediated co-transfection (Specialty Media, Inc.). Transfection was performed as described previously (Glass, et al. (1996) Cell 85:513). Flow cytometry and cell sorting were carried out on a Cytomation MoFlo (Fort Collins, CO) high speed cell sorter. Laser excitation was 130 mw at 488 nm. Fluorescence emission was collected through a 530/540 nm band pass filter for GFP. For sorting, cells were collected at a sort rate of 25,000 cells/second. The collected myoblasts were then grown to confluence, and differentiated by standard techniques into "myotubes", which are multi-nucleated muscle cells, similar to actual muscle fibers.

Expression of GFP alone did not alter the C2C12 differentiation process. Vector-driven expression of the kinase-inactive GSK3 caused a hypertrophic phenotype, resulting in thickened but shortened multinucleated myotubes. Thus, genetic manipulation of the PI3K/Akt/GSK3 pathway reveals that inhibition of GSK3 activity, mimicking the effect of activated Akt, is sufficient to cause a hypertrophic phenotype Biochemical evaluation of the PI3K/Akt/GSK3 pathways was performed by evaluating a substrate of GSK3, beta-catenin. In myotubes expressing the dominant negative, kinase-inactive form of GSK3, a decreased phosphorylation of beta-catenin was demonstrated, consistent with the blockage of endogenous GSK3 activity.

Although the foregoing invention has been described in some detail by way of illustration and example, it will be readily apparent to those of ordinary skill in the art that certain changes and modifications may be made to the teachings of the invention without departing from the spirit or scope of the appended claims.

## Claims

1. A method of identifying a test agent that inhibits muscle atrophy comprising:
(a) obtaining cells that express the following:
1) Akt;
2) an Akt reporter substrate capable of measuring Akt substrate activation;
3) Raf; and
4) a Raf reporter substrate capable of measuring Raf substrate activation;
(b) subjecting the cells to a test agent;
(c) measuring the amount of Akt substrate activation in (a)(2);
(d) measuring the amount of Raf substrate activation in (a)(4),
wherein activation of the Akt substrate absent activation of the Raf substrate is used to identify a test agent that inhibits muscle atrophy.

2. A method according to claim 1 wherein the cells are fibroblasts.

3. A method according to claim 1 or 2 wherein the cells are muscle cells.

4. A method according to claim 3 wherein the muscle cells are myoblasts.

5. A method according to claim 4 wherein the myoblasts are C2C12 cells.

6. A cell comprising:
1) Akt;
2) an Akt reporter substrate capable of measuring Akt substrate activation;
3) Raf; and
4) a Raf reporter substrate capable of measuring Raf substrate activation.

## Patentansprüche

1. Verfahren zur Identifizierung eines Testagenz, das Muskelatrophie hemmt, umfassend:
(a) Erhalten von Zellen, die folgendes exprimieren:
1) Akt;
2) ein Akt-Reportersubstrat, das in der Lage ist, Akt-Substrataktivierung zu messen;
3) Raf; und
4) ein Raf-Reportersubstrat, das in der Lage ist, Raf-Substrataktivierung zu messen;
(b) Aussetzen der Zellen einem Testagenz;
(c) Messen des Ausmaßes der Akt-Substrataktivierung in (a)(2);
(d) Messen des Ausmaßes der Raf-Substrataktivierung in (a)(4),
wobei die Aktivierung des Akt-Substrats mit ausbleibender Aktivierung des Raf-Substrats verwendet wird zur Identifizierung eines Testagenz, das Muskelatrophie verhindert.

2. Verfahren gemäß Anspruch 1, wobei die Zellen Fibroblasten sind.

3. Verfahren gemäß Anspruch loder 2, wobei die Zellen Muskelzellen sind.

4. Verfahren gemäß Anspruch 3, wobei die Muskelzellen Myoblasten sind.

5. Verfahren gemäß Anspruch 4, wobei die Myoblasten C2C12-Zellen sind.

6. Zelle, die umfasst:
1) Akt;
2) ein Akt-Reportersubstrat, das in der Lage ist, Akt-Substrataktivierung zu messen;
3) Raf; und
4) ein Raf-Reportersubstrat, das in der Lage ist, Raf-Substrataktivierung zu messen.

## Revendications

1. Méthode pour identifier un agent de test qui inhibe l'atrophie musculaire comprenant les étapes consistant à :
(a) obtenir des cellules exprimant :
1) Akt ;
2) un substrat d'Akt reporter capable de mesurer l'activation du substrat d'Akt ;
3) Raf ; et
4) un substrat de Raf reporter capable de mesurer l'activation du substrat de Raf ;
(b) soumettre les cellules à un agent de test ;
(c) mesurer la quantité d'activation du substrat d'Akt en (a) (2) ;
(d) mesurer la quantité d'activation du substrat de Raf en (a) (4),
dans laquelle l'activation de l'activation sans substrat d'Akt du substrat de Raf est utilisée pour identifier un agent de test qui inhibe l'atrophie musculaire.

2. Méthode selon la revendication 1 dans laquelle les cellules sont des fibroblastes.

3. Méthode selon la revendication 1 ou 2 dans laquelle les cellules sont des cellules musculaires.

4. Méthode selon la revendication 3 dans laquelle les cellules musculaires sont des myoblastes.

5. Méthode selon la revendication 4 dans laquelle les myoblastes sont des cellules C2C12.

6. Cellule comprenant :
1) Akt ;
2) un substrat d'Akt reporter capable de mesurer l'activation du substrat d'Akt ;
3) Raf ; et
4) un substrat de Raf reporter capable de mesurer l'activation du substrat de Raf.
